# EUROPEAN PATENT SPECIFICATION

(11) **EP 3 773 260 B1**
(45) Date of publication and mention of the grant of the patent: **28.05.2025**
(21) Application number: 19786225.3
(22) Date of filing: 10.04.2019
(51) Int. Cl.: A61B 17/16, A61B 17/56, A61B 17/70, A61F 2/44, A61F 2/30

(54) **MONOBLOCK IMPLANT**
MONOBLOCKIMPLANTAT
IMPLANT MONOBLOC

(30) Priority: 10.04.2018 US 201862655329 P
(43) Date of publication of application: 17.02.2021
(73) Proprietor: Uncinate Joint, LLC, Coeur D'Alene, ID 83814 (US)
(72) Inventor: LARSON, Jeffrey John, Coeur d'Alene, ID 83814 (US); BERTELE, Theodore P., Longmont, CO 80503 (US)
(74) Representative: Dehns Germany Partnerschaft mbB
(86) International application number: PCT/US2019/026740
(87) International publication number: WO 2019/199934

(56) References cited:
- US-A1- 2006 241 764
- US-A1- 2014 277 495
- US-A1- 2017 189 201
- US-A1- 2018 036 139
- US-A1- 2018 036 139
- US-B2- 8 617 244

## Description

### TECHNICAL FIELD

The present application relates to implants used in cervical spine (neck) fusion surgeries.

### BACKGROUND

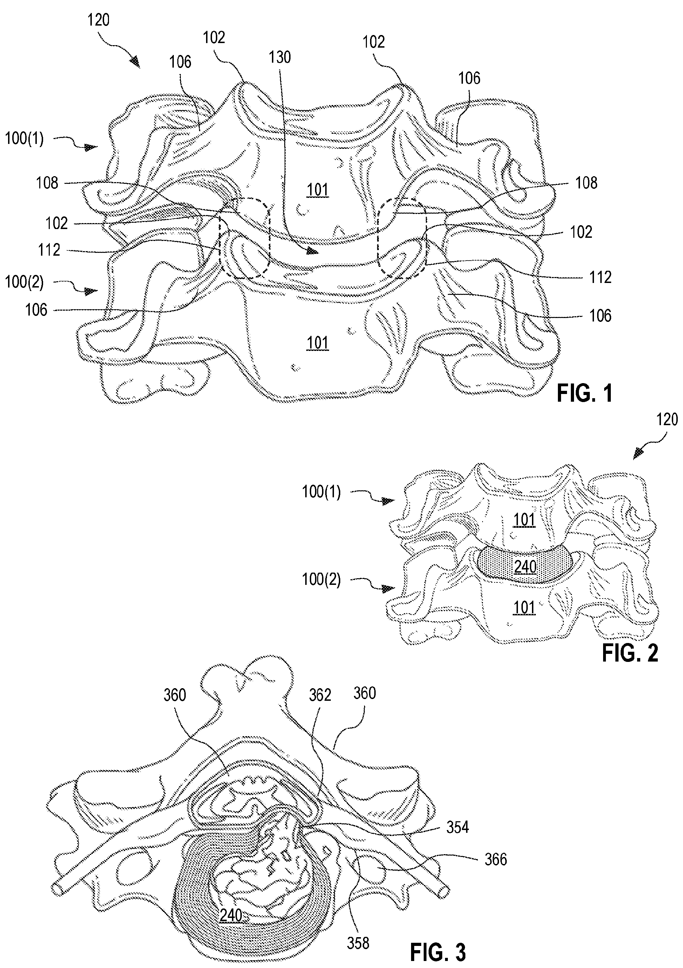

As illustrated in anterior view in FIG. 1, a typical C3 to C7 cervical vertebra 100, as found in the mid to lower neck of humans, has a mostly flat vertebral body 101 and, on each side of vertebral body 101, a vertical projection called the uncinate process 102 near where transverse processes 106 and the pedicles (not visible in FIG. 1) attach to vertebral body 101. FIG. 1 shows a superior cervical vertebra 100(1) and an inferior cervical vertebra 100(2) of a cervical motion segment 120 in the C3-C7 cervical spine. The intervertebral disk is omitted in FIG. 1, to provide a clear view of left and right uncinate processes 102 rising above vertebral body 101 of inferior vertebra 100(2). The uncinate processes 102 engage with a downward projection 108 of vertebral body 101 of superior vertebra 100(1). The joint 112 formed between uncinate process 102 of inferior vertebra 100(2) and downward projections 108 of superior vertebra 100(1) is known variously as an uncinate joint, a Luschka's joint, and an uncovertebral joint. Cervical motion segments C3/4 through C6/7 have uncinate joints 112. Between vertebral body 101 of superior vertebra 100(1) and vertebral body 101 of inferior vertebra 100(2) is an intervertebral disk space 130 (see FIG. 1). As illustrated in FIG. 2, intervertebral disk space 130 accommodates an intervertebral disk 240.

A number of ills can afflict the joints, including the uncinate joint and the intervertebral disk between cervical vertebrae causing pain and/or paralysis. For example, as illustrated in axial view in FIG. 3, the disk 240 can rupture producing a protrusion or herniation 354 projecting into the space formed by pedicles 358 and lamina 360 and within which the spinal cord 356 is located-this can apply pressure to the spinal cord 356 and associated nerve roots 362 thereby causing potentially severe neurological problems by interfering with signaling between spinal cord 356 and spinal nerves and causing pain and/or paralysis. Similarly, should protrusion or herniation 354 be located laterally, it may apply pressure to spinal nerves in foramen 366. Pressure on the nerves in foramen 366 can also cause significant neurological problems.

One treatment that has been used for ills afflicting joints between cervical vertebrae is spinal fusion.

Document US 2017/189139 A1 discloses an implant for cervical motion segment fusion, having an interbody portion and two wings. Document US 2018/036139 A1 discloses an implant for cervical motion segment fusion.

### SUMMARY

According to the invention, defined in the claims, a monoblock implant for fusing a cervical motion segment includes an interbody portion configured to be positioned between superior and inferior vertebral bodies, of the cervical motion segment, with a first side of the interbody portion facing a first uncinate joint of the cervical motion segment and a second side of the interbody portion facing a second uncinate joint of the cervical motion segment. The monoblock implant further includes a first wing extending away from the first side to extend into the first uncinate joint, and a second wing extending away from the second side to extend into the second uncinate joint.

### BRIEF DESCRIPTION OF THE DRAWINGS

FIG. 1 is an illustration of a cervical motion segment showing the uncinate joints.
FIG. 2 is an illustration of a cervical motion segment showing the intervertebral disk.
FIG. 3 is a top plan view of a cervical vertebra with disk showing spinal column, spinal nerves and nerve roots, and foramen.
FIG. 4 illustrates a monoblock implant for cervical spine fusion, according to an embodiment.
FIG. 5 shows the monoblock implant of FIG. 4 and a central portion of the cervical motion segment of FIG. 4 in further detail.
FIG. 6 illustrates a method for fusing a cervical motion segment.
FIG. 7 illustrates another method for fusing a cervical motion segment.
FIGS. 8-11 are different schematic views of the monoblock implant of FIG. 4, according to embodiments.
FIG. 12 illustrates a fusion-promoting monoblock implant, according to an embodiment.
FIG. 13 illustrates a method for promoting fusion of a cervical motion segment,.

### DETAILED DESCRIPTION OF THE EMBODIMENTS

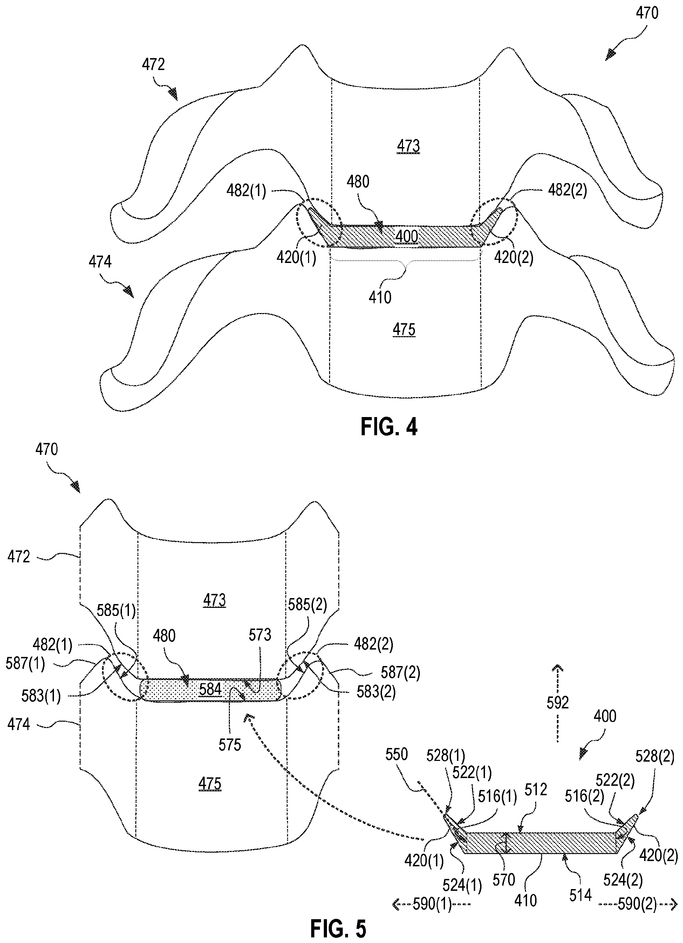

FIG. 4 illustrates one monoblock implant 400 for cervical spine fusion. FIG. 4 is an anterior view of monoblock implant 400 as implanted in a cervical motion segment 470 between a superior vertebra 472 and an inferior vertebra 474. Cervical motion segment 470 is any one of cervical motion segments C3/4, C4/5, C5/6, and C6/7. Monoblock implant 400 spans across the intervertebral disk space 480 and into both uncinate joints 482(1) and 482(2) of cervical motion segment 470. Monoblock implant 400 may be used to stabilize cervical motion segment 470 and thus treat a variety of ills afflicting cervical motion segment 470, such as neurological problems caused by pressure on the spinal cord, spinal nerve roots, spinal nerves, or spinal arteries. Monoblock implant 400 includes an interbody portion 410 and two wings 420(1) and 420(2) extending away from interbody portion 410. When implanted in cervical motion segment 470, interbody portion 410 is situated in intervertebral disk space 480, and wings 420(1) and 420(2) extend into uncinate joints 482(1) and 482(2), respectively. Monoblock implant 400 may be rigid. In one embodiment, monoblock implant 400 is a single, rigid piece.

Conventional interbody cages, used to stabilize cervical motion segments, are situated in the intervertebral disk space alone and do not extend into the uncinate joints. A conventional interbody cage is supported by the cartilaginous endplates of the vertebral bodies (e.g., superior vertebral body 473 and inferior vertebral body 475). These cartilaginous endplates are often soft, and subsidence of conventional interbody cages may therefore be significant. Such subsidence can lead to pseudoarthritis, and continued pain and neurological symptoms. In contrast, monoblock implant 400 has a substantially larger lateral footprint than a conventional interbody cage sized for the same cervical motion segment. In itself, the geometry of this larger footprint provides improved stabilization over a conventional interbody cage, and inclusion of the uncinate joints may allow for better control of cervical lordosis. In addition, the stabilization provided by monoblock implant 400 benefits from the dense cortical bone of vertebra 472 and 474 in uncinate joints 482, compared to the softer cartilaginous endplates of vertebral bodies 473 and 475 in intervertebral disk space 480. The load bearing characteristics of cortical bone is superior to that of the cartilaginous endplates of intervertebral disk space 480. As compared to a conventional interbody cage, monoblock implant 400 may therefore experience reduced subsidence. Furthermore, monoblock implant 400 has certain fusion advantages. Fusion at cortical bone is advantageous since such fusion will occur without subsidence. Thus, fusion of uncinate joints 482 via wings 420 may eliminate or reduce the subsidence issues associated with a conventional interbody cage. In addition, the presence of wings 420 for load bearing may be utilized to reduce the load bearing requirements to interbody portion 410 and thus make room for more bone graft material in intervertebral disk space 480.

FIG. 5 shows monoblock implant 400 and a central portion of cervical motion segment 470 in further detail. FIG. 5 is an anterior view of cervical motion segment 470 prior to monoblock implant 400 being inserted therein. Prior to fusion surgery, intervertebral disk space 480 typically contains an intervertebral disk 584, although intervertebral disk 584 may be damaged and/or partly missing. Intervertebral disk 584 is removed prior to insertion of monoblock implant 400 into intervertebral disk space 480.

Interbody portion 410 has two opposite-facing surfaces 512 and 514. The maximum distance between surfaces 512 and 514 defines a maximum axial extent 570 of interbody portion 410 in an axial dimension (referenced to the orientation of monoblock implant 400 when situated in cervical motion segment 470). Maximum axial extent 570 is, for example, in the range between 4 and 14 millimeters. Interbody portion 410 also has substantially opposite-facing sides 516(1) and 516(2). Wing 420(1) extends away from side 516(1), and wing 420(2) extends away from side 516(2). It is understood that sides 516 are not exposed surfaces of interbody portion 410. Rather, sides 516 indicate where monoblock implant 400 transitions between interbody portion 410 and wings 420. Each wing 420 extends (a) away from the corresponding side 516 along a lateral direction 590 and (b) upwards along a superior axial direction 592. Thus, each wing 420 is at an orientation 550 that is at an oblique angle to each of superior axial direction 592 and lateral direction 590. Each wing 520 has a surface 522 and a surface 524. Surfaces 522 and 524 may converge along the corresponding lateral direction 590, such that the thickness of wing 420 decreases with distance away from interbody portion 410. Ends 528 of wings 520 may be blunt or rounded, for example to reduce risk of ends 528 injuring structures adjacent to uncinate joints 482.

When monoblock implant 400 is implanted into intervertebral disk space 480, surface 512 faces an inferior surface 573 of superior vertebral body 473, and surface 514 faces a superior surface 575 of inferior vertebral body 475. For each wing 420, surface 522 faces an inferior surface 583 of superior vertebra 472 in uncinate joint 482, and surface 524 faces a superior, medial surface 585 of uncinate process 587 of inferior vertebra 474 in uncinate joint 482. One or more of surfaces 512, 522(1), and 522(2) of monoblock implant 400 may contact the corresponding one of surfaces 573, 583(1), and 583(2) of cervical motion segment 470, and one or more of surfaces 514, 524(1), and 524(2) of monoblock implant 400 may contact the corresponding one of surfaces 575, 585(1), and 585(2) of cervical motion segment 470, such that monoblock implant 400 defines a spacing between superior vertebra 472 and inferior vertebra 474 and bears the load between superior vertebra 472 and inferior vertebra 474. In one scenario, each of surfaces 522(1), 524(1), 522(2), and 524(2) of wings 420 contacts the corresponding surfaces 583(1), 585(1), 583(2), and 585(2), respectively, of cervical motion segment 470. In this scenario, monoblock implant 400 further provides lateral stability.

Monoblock implant 400 may cooperate with a tension band of cervical motion segment 470 to stabilize cervical motion segment 470 and, optionally, secure monoblock implant 400 in cervical motion segment 470. Herein, a "tension band" refers to a natural existing phenomenon wherein one or more existing ligaments, of cervical motion segment 470, pull vertebrae 472 and 474 toward each other, thus counteracting or balancing stretching done by monoblock implant 400 when placed between vertebrae 472 and 474.

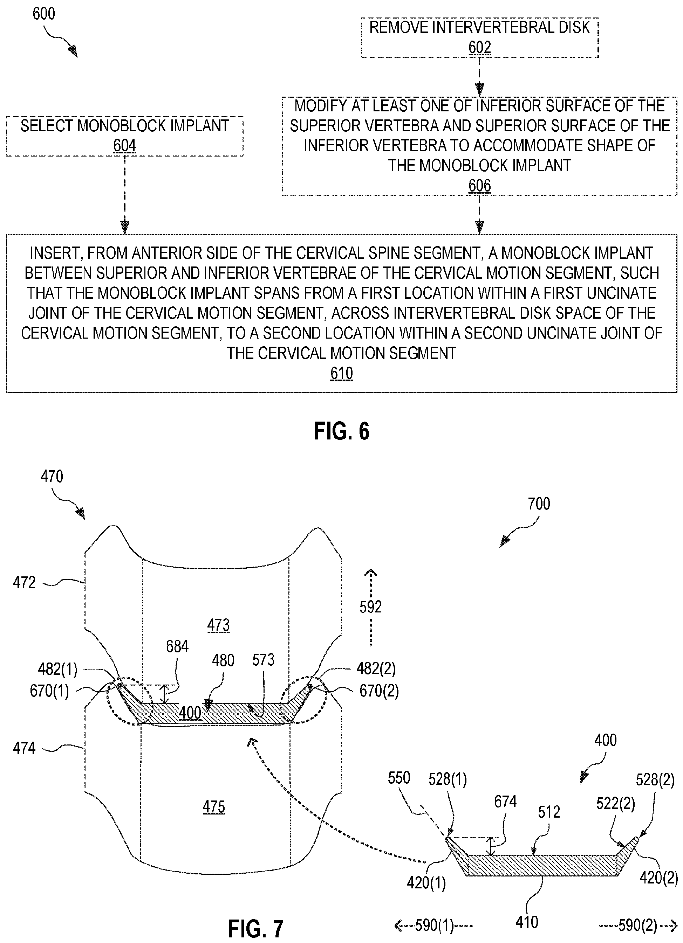

FIG. 6 is a flowchart for one method 600 (not claimed) for fusing cervical motion segment 470. Method 600 may utilize monoblock implant 400. Method 600 may be performed by a surgeon, or a robot (or another apparatus), or a combination of a surgeon and a robot (or another apparatus). Method 600 includes a step 610 of inserting, from an anterior side of cervical motion segment 470, a monoblock implant between superior vertebra 472 and inferior vertebra 474 of cervical motion segment 470. When inserted into cervical motion segment 470, the monoblock implant spans from a first location within uncinate joint 482(1), across intervertebral disk space 480, to a second location within uncinate joint 482(2). In one example of step 610, monoblock implant 400 is inserted into cervical motion segment 470 between vertebrae 472 and 474 as shown in FIG. 4.

In one embodiment, method 600 (not claimed) includes a step 602 of removing intervertebral disk 584 from intervertebral disk space 480, prior to performing step 610. Step 602 may utilize methods, known in the art, for removing an intervertebral disk.

Method 600 may further include a step 606 performed before step 610. Step 606 modifies at least one of and inferior surface of superior vertebra 472 and a superior surface of inferior vertebra 474 to accommodate the shape of the monoblock implant. Step 606 helps prepare cervical motion segment 470 for insertion of the monoblock implant. Step 606 may be performed after step 602 (as depicted in FIG. 6), in conjunction with step 602, or at least partly before step 602, without departing from the scope hereof. Step 606 may eliminate or reduce patient-to-patient anatomic variation, so as to reduce or eliminate any need for customization of the monoblock implant to match patient anatomy. In one embodiment, step 606 removes bone tissue from one or both of (a) the inferior surface of superior vertebra 472 and (b) the superior surface of inferior vertebra 474 at the interface between intervertebral disk space 480 and each of uncinate joints 482(1) and 482(2), that is, in the regions where the lateral margins of the cartilaginous endplates of vertebral bodies 473 and 475 meet the medial margins of uncinate joints 482. This embodiment of step 606 may eliminate or reduce interference between tissue of cervical motion segment 470 and portions of monoblock implant 400 at the transition regions between interbody portion 410 and wings 420. Step 606 may also include preparing intervertebral disk space 480 for receiving the monoblock implant. Step 606 may utilize methods and tools similar to those, known in the art, for removing bone tissue from other regions of a spine segment.

In an embodiment, method 600 (not claimed) includes a step 604 of selecting the monoblock implant from a kit of monoblock implants of different shapes and sizes (e.g., height, width, depth, and angles). In one example of step 604, a particular version of monoblock implant 400, characterized by a particular size and shape deemed the optimal fit for the cervical motion segment 470 under surgery, is selected from a kit of monoblock implants 400 of different shapes and sizes. In one implementation, step 604 is performed after step 606 to base the selection upon an assessment of cervical motion segment 470 after modification thereof. In another implementation, step 604 is performed before step 606, for example based upon radiological studies of cervical motion segment 470. Step 606 may then modify cervical motion segment 470 to best fit the monoblock implant selected in step 604.

FIG. 7 illustrates another method 700 (not claimed) for fusing cervical motion segment 470. Method 700 is an embodiment of step 610 of method 600. In method 700, monoblock implant 400 is inserted into cervical motion segment 470 such that monoblock implant 400 spans from location 670(1) in uncinate joint 482(1), across intervertebral disk space 480, to a location 670(2) in uncinate joint 482(2). Locations 670(1) and 670(2) are the locations of ends 528(1) and 528(2), respectively, when monoblock implant 400 is implanted into cervical motion segment 470. Each of locations 670(1) and 670(2) is superior to inferior surface 573 of superior vertebral body 473, that is, above surface 573 in the direction along superior axial direction 592 by an axial distance 684. Axial distance 684 may be the same as or similar to a distance 674 by which ends 582 are offset axially from surface 512 of interbody portion 410. Neither one of locations 670(1) and 670(2) can be reached by a flat implant that does not have wings 420. For example, a conventional interbody cage cannot reach locations 670(1) and 670(2), even if the conventional interbody cage is oversized. Each of distances 684 and 674 may be in the range between 2 and 7 millimeters.

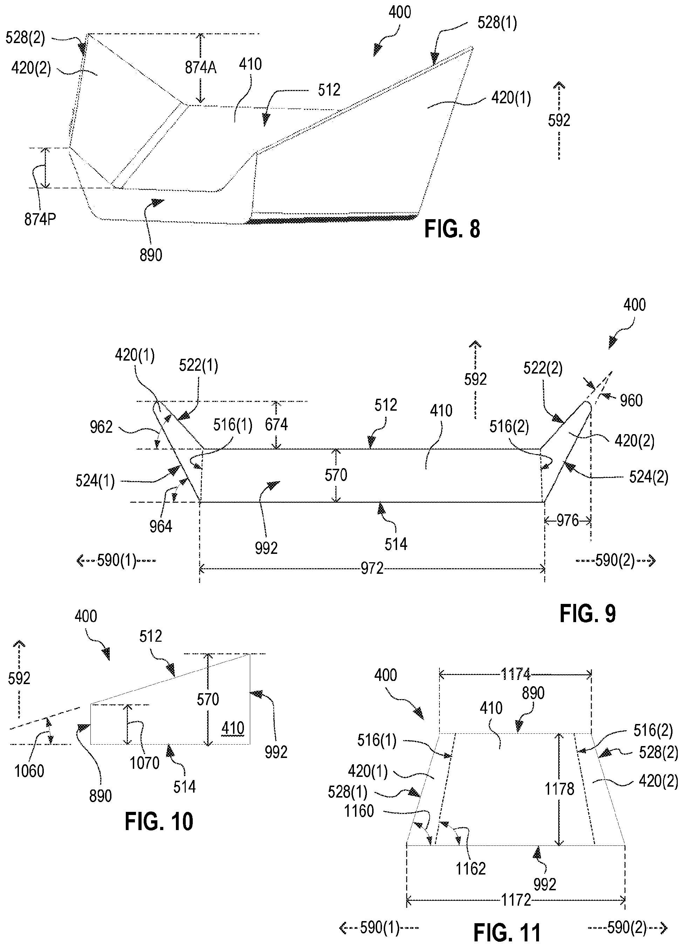

FIGS. 8-11 are different schematic views of monoblock implant 400 showing certain properties of monoblock implant in further detail. While FIGS. 8-11 depict a specific shape of monoblock implant 400, it is understood that monoblock implant 400 may take on a range of shapes that deviate from the one depicted in FIGS. 8-11 in one or more aspects. For example, each of the dimensions and angles depicted in FIGS. 8-11 may have a range of values. FIG. 8 is a perspective view of monoblock implant 400 as seen from a posterior-lateral direction. FIG. 9 is an anterior view of monoblock implant 400. FIG. 10 is a cross section of interbody portion 410 taken in a plane that is spanned by superior axial direction 592 and an anterior-to-posterior direction. FIG. 11 is an axial view of a superior side of monoblock implant 400 (viewed along a direction opposite superior axial direction 592). FIGS. 8-11 are best viewed together in the following description.

In the discussion of FIGS. 8-11, the terms anterior, posterior, superior, inferior, and lateral refer to the orientation of monoblock implant 400 when implanted in cervical motion segment, as illustrated in FIG. 4 for example. Monoblock implant 400 has an anterior side 992 (see FIG. 9) and a posterior side 890 (see FIG. 8).

In certain embodiments, monoblock implant 400 is shaped to accommodate and/or define a certain degree of lordosis. In such lordosis-corrected embodiments, the axial extent of monoblock implant 400, in dimensions parallel to superior axial direction 592, decreases along the anterior-to-posterior direction. For example, as depicted in FIG. 10, the axial extent of interbody portion 410 decreases from maximum axial extent 570, at anterior side 992, to a smaller axial extent 1070 at posterior side 890. In these embodiments, surfaces 512 and 514 are oriented at a non-zero angle 1060 to each other. Angle 1060 is, for example, in the range between zero and twenty degrees (zero not included). In the lordosis-corrected embodiments, the offset between ends 528 and surface 512 may decrease along the anterior-to-posterior direction. For example, as depicted in FIG. 8, the offset between ends 528 and surface 512 may decrease from a maximum offset 874A at the anterior extreme of monoblock implant 400 to a minimum offset 874P at posterior side 890. The decrease from maximum offset 874A to minimum offset 874P may, but need not, be characterized by angle 1060 or an angle similar thereto.

In another embodiment, monoblock implant 400 is not corrected for lordosis. In this embodiment, surfaces 512 and 514 may be parallel to each other and angle 1060 may be zero degrees. Regardless of whether or not monoblock implant 400 is corrected for lordosis, maximum axial extent 570 is, for example, in the range between 4 and 14 millimeters.

In a typical cervical motion segments 470, uncinate joints 482(1) and 482(2) converge in the anterior-to-posterior direction. That is, the distance between uncinate joints 482(1) and 482(2) decreases along the anterior-to-posterior direction. Monoblock implant 400 may be configured to match, or approximate, this anterior-to-posterior convergence of uncinate joints 482(1) and 482(2). For example, as shown in FIG. 11 (and also visible in FIG. 8), monoblock implant 400 has and anterior width 1172 and a smaller posterior width 1174. The decrease from anterior width 1172 to posterior width 1174 may be characterized by an oblique lateral angle 1160 of each end 528 relative to lateral directions 590. (Anterior side 992 may, but need not, be parallel to lateral directions 590.) Oblique lateral angle 1160 may be in the range between 45 and 90 degrees (90 degrees not included). Similarly, sides 516 may be oriented at an oblique lateral angle 1162 relatively to lateral directions 590. Oblique lateral angle 1162 may be similar to oblique angle 1160, or closer to 90 degrees. In an alternative embodiment, each of angles 1160 and 1162 is ninety degrees, and anterior width 1172 is identical to posterior width 1174.

Regardless of the value of angles 1160 and 1162, anterior width 1172 may be in the range between 15 and 32 millimeters, for example. The posterior width 972 of interbody portion 410 (see FIG. 9) may be in the range between 10 and 25 millimeters. The anterior-to-posterior extent 1178 of monoblock implant 400 (see FIG. 11) may be in the range between 10 and 20 millimeters.

Referring now to FIG. 9 in particular, each wing 420 may extend away from the corresponding side 516 of interbody portion 410 by a distance 976. Distance 976 is, for example, in the range between 3 and 7 millimeters. The shape and orientation of wings 420 may be at least partly characterized by angles 960, 962, and 964. Angle 960 is the convergence angle between surfaces 522 and 524. Angle 960 is defined such that a value of zero degrees would correspond to surfaces 522 and 524 being parallel. Angle 960 is, for example, in the range between 10 and 75 degrees or in the range between 10 and 55 degrees. Angle 962 is the angle between surface 522 and lateral directions 590. Angle 962 is, for example, in the range between 10 and 75 degrees or in the range between 30 and 75 degrees. Angle 964 is the angle between surface 524 and lateral directions 590. Angle 964 is the sum of angles 960 and 962. When surfaces 512 and 514 are parallel to lateral directions 590, angle 962 indicates the orientation of surface 522 relative to surface 512, and angle 964 indicates the orientation of surface 524 relative to surface 514.

Although FIG. 9 shows sharp corners between interbody portion 410 and wings 420, these corners may be rounded, for example as depicted in FIG. 8. All other sharp edges depicted in FIGS. 8-11 may be rounded as well, without departing from the scope hereof.

A kit may include multiple different shapes and sizes of monoblock implant 400. For example, a kit may include different combinations of at least some of the distance-type dimensions and angle-type dimensions depicted in FIGS. 8 and 11. In such a kit, distance-type dimensions may be incremented by one or several millimeters between adjacent sizes. Anterior width 1172 and anterior-to-posterior extent 1178 may be incremented in steps of 2-5 millimeters. Maximum axial extent 570, and optionally also distance 674, may be incremented in steps of 1-3 millimeters. One or both of angles 1060 and 1160 may be incremented in steps of 5-15 degrees. Angle 960 may be the same for all instances of monoblock implant 400 in a kit, or incremented in steps of 1-5 degrees.

Although each of surfaces 512, 514, 522(1), 524(1), 522(2), and 524(2) are shown herein as being planar, one or more of these surfaces may be at least slightly curved without departing from the scope hereof.

Monoblock implant 400 may be substantially composed of a metal such as titanium, titanium alloy, stainless steel, cobalt, chromium, or a combination thereof. Without departing from the scope hereof, such metal embodiments of monoblock implant 400 may include a coating, for example a hydroxyapatite coating, to achieve improved fixation of monoblock implant 400 in cervical motion segment 470. In another embodiment, monoblock implant 400 includes a porous portion with pores capable of accommodating bone graft material. In one example hereof, monoblock implant 400 is substantially composed of, or includes, porous metal. In a related embodiment, at least a portion of monoblock implant 400 is a porous portion substantially composed of bone graft material. In yet another embodiment, monoblock implant 400 is substantially composed of allograft bone. In another embodiment, monoblock implant 400 includes a polymer, such as polyetheretherketone (PEEK) or another polyaryletherketone (PAEK) polymer. Any of the above materials may be used in a 3-D printing process to make monoblock implant 400.

Although not shown in FIGS. 8-12, one or more of surfaces 512, 514, 522(1), 524(1), 522(2), and 524(2) may form features (e.g., ribs, barbs, rough surface, and/or sawtooth shaped features) that help secure monoblock implant 400 to vertebrae 472 and 474.

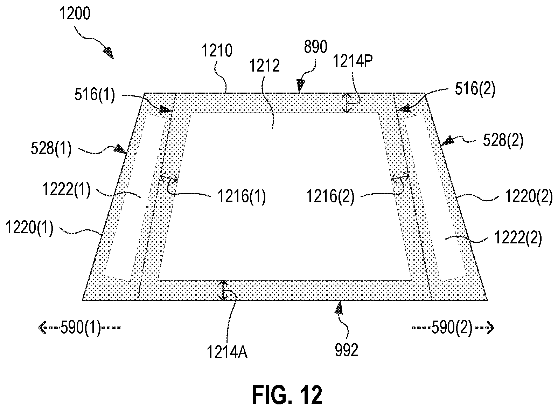

FIG. 12 illustrates one fusion-promoting monoblock implant 1200. FIG. 12 is an axial view of the superior side of monoblock implant 1200. Monoblock implant 1200 is an embodiment of monoblock implant 400 and may take on any shape and size discussed above in reference to monoblock implant 400. For example, although FIG. 12 shows monoblock implant 1200 with end 528(1) and 528(2) converging in the anterior-to-posterior direction from anterior side 992 to posterior side 890, ends 528(1) and 528(2) may instead be parallel. Monoblock implant 1200 includes an interbody portion 1210 and wings 1220(1) and 1220(2), embodiments of interbody portion 410 and wings 420(1) and 420(2), respectively.

Interbody portion 1210 forms at least one void 1212 for carrying bone graft material into intervertebral disk space 480. FIG. 12 shows a single void 1212, but interbody portion 1210 may form multiple voids 1212 without departing from the scope hereof; for example, the single void 1212 depicted in FIG. 12 may be split in two with an anterior-to-posterior supporting bridge between the two voids. Generally, each void 1212 may extend through the entire axial extent of interbody portion 1210. For structural integrity of monoblock implant 1200, the single void 1212 or the collection of multiple voids 1212 is surrounded by a material part of monoblock implant 1200. On the anterior side of void(s) 1212, the anterior-to-posterior thickness of the material part of interbody portion 1210 has a minimum thickness 1214A. Minimum thickness 1214A may be constant along lateral directions 590, as shown in FIG. 12, or exhibit variation along lateral directions 590. Similarly, on the posterior side of void(s) 1212, the anterior-to-posterior thickness of the material part of interbody portion 1210 has a minimum thickness 1214P. Minimum thickness 1214P may be constant along lateral directions 590, as shown in FIG. 12, or exhibit variation along lateral directions 590. Laterally to void(s) 1212, interbody portion 1210 may have material parts characterized by a minimum thickness 1216. Alternatively, void(s) 1212 may span all the way from side 516(1) to side 516(2), corresponding to minimum thickness 1216 being zero, and the structural integrity is instead provided by wings 1220. The presence of wings 1220 thereby allows interbody portion 1210 to carry more bone graft material than what can be carried by a conventional interbody cage. In one example, void(s) 1212 occupy at least 45 percent or at least 90 percent of the volume of the envelope spanned by interbody portion 1210 within sides 516(1), 516(2), 890, and 992. In another example, void(s) 1212 occupy between 70 and 90 percent of the volume of the envelope spanned by interbody portion 1210 within sides 516(1), 516(2), 890, and 992.

Each wing 1220 may also form one or more voids 1222 for carrying bone graft material into the respective uncinate joint 482. Each void 1222 may extend all the way through the corresponding wing 1220 between surfaces 522 and 524 (see FIG. 5). In an alternative embodiment, interbody portion 1210 does not form any voids 1212, but each wing 1220 forms at least one void 1222. However, to optimize the stabilization provided by monoblock implant 1200, especially the degree of cervical lordosis control, it may be advantageous that wings 1220 form no voids 1222, or that any voids 1222 formed by wings 1220 occupy only a small portion of the envelopes spanned by wings 1220.

In certain embodiments, wings 1220 include, or are composed of, a porous material to allow bone growth through wings 1220 to fuse uncinate joints 482.

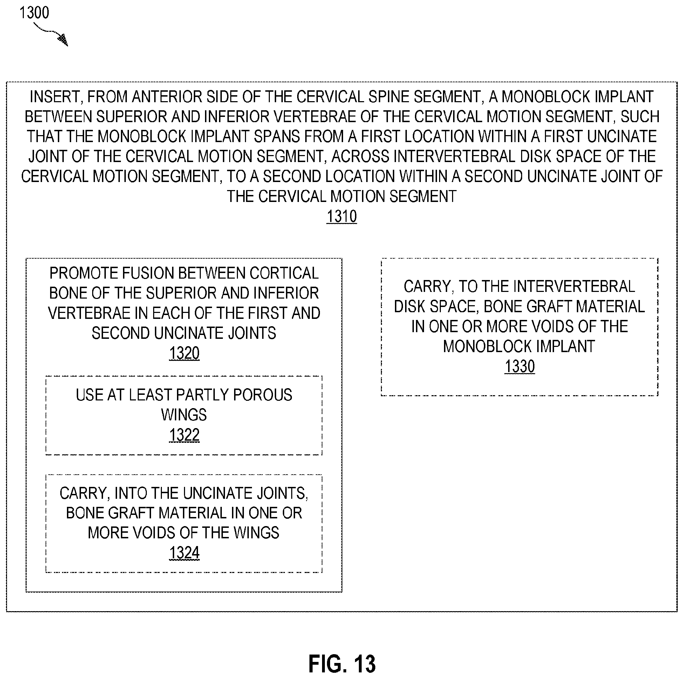

FIG. 13 is a flowchart for one method 1300 method 1300 (not claimed) for promoting fusion of cervical motion segment 470. Method 1300 is an embodiment of step 610 of method 600. Method 1300 includes a step 1310 inserting, from an anterior side of cervical motion segment 470, a monoblock implant between superior vertebra 472 and inferior vertebra 474 of cervical motion segment 470. When inserted into cervical motion segment 470, the monoblock implant spans from a first location within uncinate joint 482(1), across intervertebral disk space 480, to a second location within uncinate joint 482(2). Step 1310 includes a step 1320 of promoting fusion between cortical bone of the superior vertebra 472 and inferior vertebra 474 in each of uncinate joints 482(1) and 482(2). In one example of steps 1310 and 1320, monoblock implant 400 is inserted into cervical motion segment 470, wherein each wing 420 of monoblock implant 400 is configured to promote fusion.

In one embodiment, step 1320 implements a step 1322 of using at least partly porous wings. For example, each wing 420 of monoblock implant 400 may include or be composed of a porous material, as discussed above in reference to FIGS. 8-12. In another implementation, step 1320 implements a step 1324 of carrying, into uncinate joints 482, bone graft material in one or move voids of the wings. For example, each wing 420 may form one or more voids for carrying bone-graft material into the respective uncinate joint 482, as discussed above in reference to FIG. 12 (see voids 1222). In yet another embodiment, step 1320 implements both step 1322 and 1324. For example, each wing 420 may include or be composed of a porous material, and further form one or more voids for carrying bone graft material. Alternatively, or in combination with one or both of steps 1322 and 1324, each wing may be at least partly composed of allograft bone.

Step 1310 may further include a step 1330 of carrying, to intervertebral disk space 480, bone graft material in one or more voids of the monoblock implant. For example, step 1310 may utilize monoblock implant 1200 forming one or more voids 1212 in interbody portion 1210.

While the invention has been particularly shown and described with reference to a preferred embodiment thereof, it will be understood by those skilled in the art that various other changes in the form and details may be made without departing from the scope of the invention. It is to be understood that various changes may be made in adapting the invention to different embodiments without departing from the broader inventive concepts disclosed herein and comprehended by the claims that follow.

## Claims

1. A monoblock implant (400) for fusing a cervical motion segment (470), comprising:
an interbody portion (410) configured to be positioned between superior and inferior vertebral bodies, of the cervical motion segment, with a first side of the interbody portion facing a first uncinate joint (482(1)) of the cervical motion segment and a second side of the interbody portion facing a second uncinate joint (482(2)) of the cervical motion segment;
a first wing (420(1)) extending away from the first side to extend into the first uncinate joint; and
a second wing (420(2)) extending away from the second side to extend into the second uncinate joint;
**characterized by** the interbody portion including a superior interbody surface (512) configured to face the superior vertebral body and an inferior interbody surface (514) configured to face the inferior vertebral body, maximum distance between the superior and inferior interbody surfaces defining an axial extent (570) of the interbody portion (410) in an axial dimension, each of the first (420(1)) and second wings (420(2)) being oriented at an oblique angle to the axial extent (570) to reach beyond the superior interbody surface (512) for a distance in the axial dimension (592); and
wherein the distance by which each of the first and second wings extends beyond the superior interbody surface (512) decreases along an anterior-to-posterior direction.

2. The monoblock implant of claim 1, being rigid.

3. The monoblock implant of claim 1, the axial extent decreasing along an anterior-to-posterior direction.

4. The monoblock implant of any of the preceding claims, each of the first and second wings including:
a superior wing surface configured to face superior vertebra of the cervical motion segment, the superior wing surface being oriented at a first oblique angle, in range between 10 and 75 degrees, to the superior interbody surface; and
an inferior wing surface configured to face inferior vertebra of the cervical motion segment, the inferior wing surface being oriented at a second oblique angle to the inferior interbody surface, the second oblique angle being at least as large as the first oblique angle, particularly the superior wing surface forming a continuation of the superior interbody surface, the inferior wing surface forming a continuation of the inferior interbody surface.

5. The monoblock implant of any of the preceding claims, each of the first and second wings including:
a superior wing surface configured to face superior vertebra of the cervical motion segment, the superior wing surface being oriented at an oblique angle to the superior interbody surface; and
an inferior wing surface configured to face inferior vertebra of the cervical motion segment, the inferior wing surface being oriented at an oblique angle to the inferior interbody surface;
distance between the superior and inferior wing surfaces decreasing with distance from the interbody portion, particularly for each of the first and second wings, the superior and inferior wing surfaces converging at a rounded edge farthest from the interbody portion.

6. The monoblock implant of any of the preceding claims, the first and second sides being non-parallel such that lateral width of the interbody portion decreases along an anterior-to-posterior direction, or at least part of each of the first and second wings being porous.

7. The monoblock implant of any of the preceding claims, forming, in at least one of (a) the interbody portion and (b) each of the first and second wings, one or more voids for accommodating a fusion promoting material.

8. The monoblock implant of claim 7, at least one of the voids being formed by the interbody portion and occupying at least 90 percent of volume of envelope spanned by the interbody portion.

## Patentansprüche

1. Monoblock-Implantat (400) zur Fixierung bzw. Fusionierung eines zervikalen Bewegungssegments (470), umfassend:
einen Zwischenkörperabschnitt (410), der so konfiguriert ist, dass er zwischen oberen und unteren Wirbelkörpern des zervikalen Bewegungssegments positioniert werden kann, wobei eine erste Seite des Zwischenkörperabschnitts einem ersten uncinaten bzw. hakenförmigen Gelenk (482(1)) des zervikalen Bewegungssegments zugewandt ist und eine zweite Seite des Zwischenkörperabschnitts einem zweiten uncinaten bzw. hakenförmigen Gelenk (482(2)) des zervikalen Bewegungssegments zugewandt ist;
einen ersten Flügel (420(1)), der sich von der ersten Seite weg erstreckt, um sich in das erste uncinate Gelenk zu erstrecken; und
einen zweiten Flügel (420(2)), der sich von der zweiten Seite weg erstreckt, um sich in das zweite uncinate Gelenk zu erstrecken;
**dadurch gekennzeichnet, dass** der Zwischenkörperabschnitt eine obere Zwischenkörperfläche (512) aufweist, die so konfiguriert ist, dass sie dem oberen Wirbelkörper zugewandt ist, und eine untere Zwischenkörperfläche (514), die so konfiguriert ist, dass sie dem unteren Wirbelkörper zugewandt ist, der maximale Abstand zwischen der oberen und der unteren Zwischenkörperoberfläche eine axiale Ausdehnung (570) des Zwischenkörperabschnitts (410) in einer axialen Dimension definiert, wobei jeder der ersten (420(1)) und zweiten Flügel (420(2)) in einem schrägen Winkel zu der axialen Ausdehnung (570) ausgerichtet ist, um über die obere Zwischenkörperoberfläche (512) über eine Distanz in der axialen Dimension (592) hinaus zu reichen; und
wobei der Abstand, um den sich sowohl der erste als auch der zweite Flügel über die obere Zwischenkörperfläche (512) hinaus erstreckt, entlang einer Richtung von anterior nach posterior abnimmt.

2. Monoblock-Implantat nach Anspruch 1, das starr ist.

3. Monoblock-Implantat nach Anspruch 1, wobei die axiale Ausdehnung entlang einer Richtung von anterior nach posterior abnimmt.

4. Monoblock-Implantat nach einem der vorhergehenden Ansprüche, wobei jeder der ersten und zweiten Flügel einschließt:
eine obere Flügelfläche, die so konfiguriert ist, dass sie dem oberen Wirbel des zervikalen Bewegungssegments zugewandt ist, wobei die obere Flügelfläche in einem ersten schrägen Winkel im Bereich zwischen 10 und 75 Grad zu der oberen Zwischenkörperfläche ausgerichtet ist; und
eine untere Flügelfläche, die so konfiguriert ist, dass sie dem unteren Wirbel des zervikalen Bewegungssegments zugewandt ist, wobei die untere Flügelfläche in einem zweiten schrägen Winkel zu der unteren Zwischenkörperfläche ausgerichtet ist, wobei der zweite schräge Winkel mindestens so groß ist wie der erste schräge Winkel, wobei insbesondere die obere Flügelfläche eine Fortsetzung der oberen Zwischenkörperfläche bildet und die untere Flügelfläche eine Fortsetzung der unteren Zwischenkörperfläche bildet.

5. Monoblock-Implantat nach einem der vorhergehenden Ansprüche, wobei jeder der ersten und zweiten Flügel einschließt:
eine obere Flügelfläche, die so konfiguriert ist, dass sie dem oberen Wirbel des zervikalen Bewegungssegments zugewandt ist, wobei die obere Flügelfläche in einem schrägen Winkel zu der oberen Zwischenkörperfläche ausgerichtet ist; und
eine untere Flügelfläche, die so konfiguriert ist, dass sie dem unteren Wirbel des zervikalen Bewegungssegments zugewandt ist, wobei die untere Flügelfläche in einem schrägen Winkel zur unteren Zwischenkörperfläche ausgerichtet ist;
wobei der Abstand zwischen den oberen und unteren Flügelflächen mit der Entfernung vom Zwischenkörperteil abnimmt, insbesondere für jeden der ersten und zweiten Flügel, wobei die oberen und unteren Flügelflächen an einer abgerundeten Kante konvergieren, die am weitesten vom Zwischenkörperteil entfernt ist.

6. Monoblock-Implantat nach einem der vorhergehenden Ansprüche, wobei die erste und die zweite Seite nicht parallel sind, so dass die seitliche Breite des Zwischenkörperteils entlang einer Richtung von anterior nach posterior abnimmt, oder mindestens ein Teil jedes der ersten und zweiten Flügel porös ist.

7. Monoblock-Implantat nach einem der vorhergehenden Ansprüche, das in mindestens einem von (a) dem Zwischenkörperteil und (b) jedem der ersten und zweiten Flügel einen oder mehrere Hohlräume zur Aufnahme eines fusionsfördernden Materials bildet.

8. Monoblock-Implantat nach Anspruch 7, wobei mindestens einer der Hohlräume von dem Zwischenkörperteil gebildet wird und mindestens 90 Prozent des Volumens der von dem Zwischenkörperteil aufgespannten Umhüllung einnimmt.

## Revendications

1. Un implant monobloc (400) pour la fusion d'un segment de mouvement cervical (470), comprenant :
une partie intersomatique (410) configurée pour être positionnée entre les corps vertébraux supérieur et inférieur du segment de mouvement cervical, avec un premier côté de la partie intersomatique faisant face à une première articulation uncinate (482(1)) du segment de mouvement cervical et un second côté de la partie intersomatique faisant face à une seconde articulation uncinate (482(2)) du segment de mouvement cervical ;
une première aile (420(1)) s'éloignant du premier côté pour s'étendre dans la première articulation uncinate ; et
une deuxième aile (420(2)) s'éloignant du deuxième côté pour s'étendre dans la deuxième articulation uncinate ;
**caractérisée par le fait que** la partie intersomatique comprend une surface intersomatique supérieure (512) configurée pour faire face au corps vertébral supérieur et une surface intersomatique inférieure (514) configurée pour faire face au corps vertébral inférieur, la distance maximale entre les surfaces intersomatiques supérieure et inférieure définissant une étendue axiale (570) de la partie intersomatique (410) dans une dimension axiale, chacune de la première (420(1)) et de la seconde aile (420(2)) étant orientée à un angle oblique par rapport à l'étendue axiale (570) pour dépasser la surface intersomatique supérieure (512) sur une distance dans la dimension axiale (592) ; et
dans lequel la distance à laquelle chacune des première et deuxième ailes s'étend au-delà de la surface intersomatique supérieure (512) diminue le long d'une direction antérieure à postérieure.

2. L'implant monobloc de la revendication 1 qu'est rigide.

3. L'implant monobloc de la revendication 2, l'étendue axiale diminuant le long d'une direction antérieure à postérieure.

4. L'implant monobloc de l'une quelconque des revendications précédentes, chacune des première et deuxième ailes comprenant :
une surface d'aile supérieure configurée pour faire face à la vertèbre supérieure du segment de mouvement cervical, la surface d'aile supérieure étant orientée à un premier angle oblique, compris entre 10 et 75 degrés, par rapport à la surface intersomatique supérieure ; et
une surface d'aile inférieure configurée pour faire face à la vertèbre inférieure du segment de mouvement cervical, la surface d'aile inférieure étant orientée à un second angle oblique par rapport à la surface intersomatique inférieure, le second angle oblique étant au moins aussi grand que le premier angle oblique, en particulier la surface d'aile supérieure formant un prolongement de la surface intersomatique supérieure, la surface d'aile inférieure formant un prolongement de la surface intersomatique inférieure.

5. L'implant monobloc de l'une quelconque des revendications précédentes, chacune des première et deuxième ailes comprenant :
une surface d'aile supérieure configurée pour faire face à la vertèbre supérieure du segment de mouvement cervical, la surface d'aile supérieure étant orientée à un angle oblique par rapport à la surface intersomatique supérieure ; et
une surface d'aile inférieure configurée pour faire face à la vertèbre inférieure du segment de mouvement cervical, la surface d'aile inférieure étant orientée à un angle oblique par rapport à la surface intersomatique inférieure ;
la distance entre les surfaces supérieure et inférieure de l'aile diminue à mesure que l'on s'éloigne de la partie du corps, en particulier pour chacune des première et deuxième ailes, les surfaces supérieure et inférieure de l'aile convergeant vers un bord arrondi le plus éloigné de la partie du corps.

6. L'implant monobloc de l'une quelconque des revendications précédentes, le premier et le second côtés n'étant pas parallèles de sorte que la largeur latérale de la partie intersomatique diminue le long d'une direction antérieure à postérieure, ou au moins une partie de la première et de la seconde aile étant poreuse.

7. L'implant monobloc de l'une quelconque des revendications précédentes, formant, dans au moins l'une (a) de la partie intersomatique et (b) de la première et de la deuxième aile, un ou plusieurs vides destinés à accueillir un matériau favorisant la fusion.

8. L'implant monobloc de la revendication 7, au moins un des vides étant formé par la partie intersomatique et occupant au moins 90 % du volume de l'enveloppe couverte par la partie intersomatique.
